# EUROPEAN PATENT APPLICATION

(11) **EP 1 854 495 A1**
(43) Date of publication of application: **14.11.2007**
(21) Application number: 05781828.8
(22) Date of filing: 31.08.2005
(51) Int. Cl.: A61M 25/00, A61M 31/00

(54) **APPARATUS FOR DELIVERY OF A MEDIA INTO A BODY CAVITY AND APPARATUS FOR BLOCKING ULTRASOUND**

(30) Priority: 31.01.2005 CN 200510004987
(71) Applicant: CHONGQING HAIFU(HIFU)TECHNOLOGY CO., LTD, Chongqing 401121 (CN)
(72) Inventor: WANG, Zhilong, 401121 Chongqing (CN); WANG, Zhibiao, 401121 Chongqing (CN); BAI, Jin, 401121 Chongqing (CN); XIONG, Zhengai, 401121 Chongqing (CN)
(74) Representative: Kampfenkel, Klaus
(86) International application number: PCT/CN2005/001368
(87) International publication number: WO 2006/079267

(57) **Abstract**

The present invention generally relates to a medical apparatus. More particularly, the present invention relates to an apparatus for delivering a medium to body cavity of mammals including a human, especially to an apparatus for expanding rectum. Specifically, the present invention relates to the field of focused ultrasound treatment with a main purpose to provide an auxiliary device for ultrasound therapy applied to uterine myoma so as to avoid damage by ultrasound after focused within body. Therefore, the present invention provides an apparatus for blocking ultrasound, comprising (1) lead-in means for entering into said body cavity; and (2) accommodation means for containing ultrasound blocking medium, which enwraps the lead-in means outside.

## Description

### Field of the Invention

The present invention generally relates to an apparatus for diagnosis and treatment. More particularly, the present invention relates to an apparatus for delivering a medium to body cavity of mammals including a human. Especially, the present invention relates to an apparatus for leading in and containing medium in a body cavity of mammals, particularly rectum. Also, the present invention relates to an apparatus for expanding a body cavity of mammals, particularly rectum. Meanwhile, the present invention relates to the field of focused ultrasound treatment, specifically, the present invention relates to an apparatus for blocking ultrasound within body.

### Background of the Invention

There are many kinds of devices for delivering a medium to a body cavity of mammals in the existing technologies. Generally, they are designed to lead in some medium, for example, a contrast agent, which is integrated with some tissue and absorbed by some tissue structure. Additionally, in most of the cases, the medicine or nutriments are led in and the purpose is mainly to deliver some medium for rinsing, activating or administrating medicine and providing nutriments. Generally, this kind of devices cannot maintain and accommodate the medium, also have no function to make a block between the medium and the tissue of animal, so it can't prevent the body cavity from being stimulated by the medium. In other words, for the delivery devices of the existing technologies, normally the part of the device that is placed into the body is open or semi-open or has an infiltration structure. For a special treatment and diagnosis, the device with a main purpose of occupying position to lead in medium so as to realize expanding, blocking or making treatment convenient has not been disclosed.

In recent years, the scope of using ultrasound therapy has become wide increasingly. With the development of ultrasound therapeutic techniques, the special acoustic therapy, in particular the high-intensity focused ultrasound (use of damaging dose) has been applied to treat many kinds of diseases, for example, the calculus, tumor and etc. By use of good penetrability and orientation of ultrasound waves, the ultrasound waves outside are focused to a point inside human or animal body. Then, the mechanical, thermal and cavitation effects at the focal point are utilized to treat diseases.

High-intensity focused ultrasound (HIFU) therapy is to focus the ultrasound waves with a relatively low average intensity level (normally several Watts per square centimeter) emitted from the ultrasound applicator at a spatial focal spot and a focal region with an average acoustic intensity level of over 1000W/cm² is formed. Consequently the temperature at the focal region instantaneously (0.1 to 5 seconds) has a sharp rise (normally beyond 70 degrees centigrade). Accordingly the tissue at the focal region can be damaged and the therapy is achieved.

When the ultrasound waves are transmitted from one medium to another medium, one part of these waves are reflected back to the first medium at the interface of two mediums (reflection) and the rest permeates the interface and goes through the second medium, but its transmission direction may deflect (refraction). The degree of acoustic reflection at the interface depends on acoustical impedance difference between the two mediums. Big difference brings a big reflection (the product of density of medium and the acoustic speed refers to acoustical impedance of medium). The reflection between fat and muscle is less, while the reflection on the bone surface is much more. Almost 100% of reflection occurs at the interface between the acoustic applicator and the air. The strong reflections may cause a lot of energy deposition and bring unexpected energy release or energy deposition at the place beyond the focal point.

In the field of clinical uses of high-intensity focused ultrasound, due to complex anatomical structures and tissue characteristics and for different treatment purposes and treatment positions, specific technical means is needed to control the transmission of ultrasound waves so as to realize the expected focusing and meanwhile avoid the damage to the non-targeted tissue in the acoustic transmission way, and consequently, the good therapeutic effects can be achieved and the safe treatment can be ensured.

There are a lot of specific problems in the study on clinical uses of high-intensity focused ultrasound. One of the developing trends of HIFU therapy is to provide and improve the treatment protocols for various kinds of treatment positions and accordingly such appropriate therapeutic equipments are provided.

For example, when the HIFU therapy is applied to uterine myoma, the ultrasound waves are transmitted in from lower abdomen and the focal point is located within the target area of uterus, and most of the acoustic energy can be deposited at the focal point, but still small part of acoustic energy can further transmit forward beyond the focal point. When the ultrasound waves go through the rectum and if there is any air in the rectum, the ultrasound waves may be reflected between the air and the rectal wall and accordingly the energy deposition occurs and the rectal wall beside the rectum is damaged and even the rectal wall is pierced. If there is no any air in the rectum, the ultrasound waves may go through the rectum. When the ultrasound waves arrive at spinal column, because an interface with a big acoustical impedance difference is formed between the bone and the muscle and nerve tissue, a strong reflection and absorption may occur at the surface close to the spinal column and a lot of energy deposition may be produced and an obvious lesion point is formed. It is well-known that there are abundant nerves distributed around the spinal column and the nervous system is more sensitive to the ultrasound wave and the damage caused by it. So, the patient to be treated may be brought quite serious side effects easily, even the paralysis of body below that lesion point may be caused.

During the treatment as above mentioned, in order to improve the safety of the treatment, how to remove the air in the rectum and how to stop the emanative ultrasound waves going through the rectum are the suspending problems for the existing technologies.

The existing technologies do not disclose any ultrasound blocking means or the relative technical solution in the subject to be treated and also do not disclose how to overcome the said difficulties existed in the treatment of uterine myoma by ultrasound therapy.

### Summary of the Invention

The first problem to be solved by the present invention is to provide an apparatus for delivering a medium to body cavity of mammals. It can enter into the body cavity and accommodate and contain a medium. It can keep a certain shape and accordingly the purposes of occupying, expanding, blocking and convenient treatment can be realized.

The second problem to be solved by the present invention is to provide an apparatus with a special structure for expanding a body cavity of mammal, further the apparatus is particularly adaptive to lead a flexible vesicle into the body cavity of mammal without damage to the body cavity.

The third problem to be solved by the present invention is to provide an apparatus for blocking ultrasound. By use of the above-mentioned apparatus for expanding a body cavity, the medium is delivered in so as to solve the problem of the damage to the subject to be treated in a certain transmission way after the ultrasound waves are focused within body.

In order to solve the above mentioned technical problems, the technical solutions provided by the present invention are as follows:

Firstly, the present invention provides an apparatus for delivering a medium to body cavity of mammals. This apparatus comprises: (1) lead-in means for entering into the body cavity of mammals; (2) accommodation means for containing the medium, which enwraps the lead-in means outside.

The lead-in means as above mentioned may open and close. It enters into the body cavity in a closing status and then expands and maintains a certain shape. When that apparatus is drawn out, it shall close. A connecting rod structure may be adopted to realize the expanding and closing. The connecting rod structure of the lead-in means may adopt nuts and screws and can be operated manually. Certainly, it can be operated through the push method known by the technicians skilled in this art. But the push force shall be controlled carefully to avoid the damage to the tissue.

This apparatus may further comprise medium delivering means. It may be integrated with the medium delivering means or it may be connected to the medium delivering means by way of plug-in. Additionally, this apparatus may also comprise medium storing means for storing the medium before use. The medium storing means may be integrated with the medium delivering means. For example, the reserve vesicle is adopted to deliver the medium by pressing it to the device with a single reverse mechanism. Accommodation means may be the vesicle made of flexible material.

According to different purposes of use, the medium can be selected from blocker, absorbing and attenuating agent.

If the ultrasound therapy needs an ultrasound block within body, the medium is selected from ultrasound attenuating agent and may be selected from the group consisting of free castor oil, silicone oil, soybean oil and peanut oil.

The apparatus as above mentioned may be used for mammals including human. It is especially suitable for delivering the medium to the rectum and accommodating the medium so as to meet the demands, for example, removal of air in rectum, blocking continuous transmission of ultrasound by medium accommodated in rectum.

The present invention also provides an apparatus for expanding a body cavity of mammal. The lead-in means with a connecting rod structure may be adopted to realize its expanding and closing. It is particularly adaptive to expand the rectum of mammal.

The nuts and screws may be adopted to operate the connecting rod structure manually so as to realize its expanding and closing. Also, the vesicle made of flexible material may be led into said body cavity.

Also, the present invention provides an apparatus for blocking ultrasound. This apparatus comprises: (1) lead-in means for entering into the body cavity of mammals; (2) accommodation means for containing the medium, which enwraps the lead-in means outside and is used to block the ultrasound.

The accommodation means of the apparatus for blocking ultrasound may be the vesicle made of flexible material. The lead-in means may open or close. This lead-in means adopts a connecting rod structure to realize the expanding and closing. Wherein said body cavity within mammals is preferably the rectum. The connecting rod structure of the lead-in means may adopt nuts and screws and can be operated manually.

The present invention provides a novel apparatus for delivering a medium to the body cavity of mammals. It is quite different from the traditional delivering device. Its accommodation structure is closed and may accommodate different kinds of mediums so as to realize occupying, expanding body cavity, achieving protective block in treatment or making treatment convenient. The apparatus disclosed in the present invention can also be used for blocking in other radiological treatments.

The expanding apparatus disclosed in the present invention can achieve expanding gradually and has an accurate, controllable and full expanding degree with less pain without anaesthesia, laceration of intestinal wall, bleeding and intestinal fistula. It has a simple manufacturing process and a low cost.

Particularly, the present invention provides a simple and effective auxiliary device for special ultrasound therapy, in particular the treatment of uterine myoma. It can apply the ultrasound attenuating agent to occupy the rectum of the patient and accordingly it makes the ultrasound transmission way free of air and absorbs and attenuates the ultrasound energy behind the focal point so as to avoid bringing a lesion point on the surface of spinal column. The present invention has solved the difficulties in the high-intensity focused ultrasound therapy applied to uterine myoma and ensures a safe HIFU therapy to a patient. Therefore, a non- traumatic or non-invasive treatment can be realized, the treatment cost for a patient can be reduced greatly, the treatment time can be shortened and the pain can be reduced. Consequently, the apparatus brings good social benefits.

### BRIEF DESCRIPTION OF THE FIGURES

After the summary of the invention, the objects and features of the invention can be better understood by the technicians skilled in this art with reference to the following detailed description and accompanying drawings.
Fig. 1 is a graph illustrating the high-intensity focused ultrasound therapy applied to uterine myoma.
Fig. 2 is a graph illustrating the closing status of the apparatus in accordance with the present invention.
Fig. 3 is a graph illustrating the expanding status of the apparatus in accordance with the present invention showed in Fig. 2.
Fig. 4 is a magnifying graph for the handle of the apparatus in accordance with the present invention.

### DETAILED DESCRIPTION OF THE INVENTION

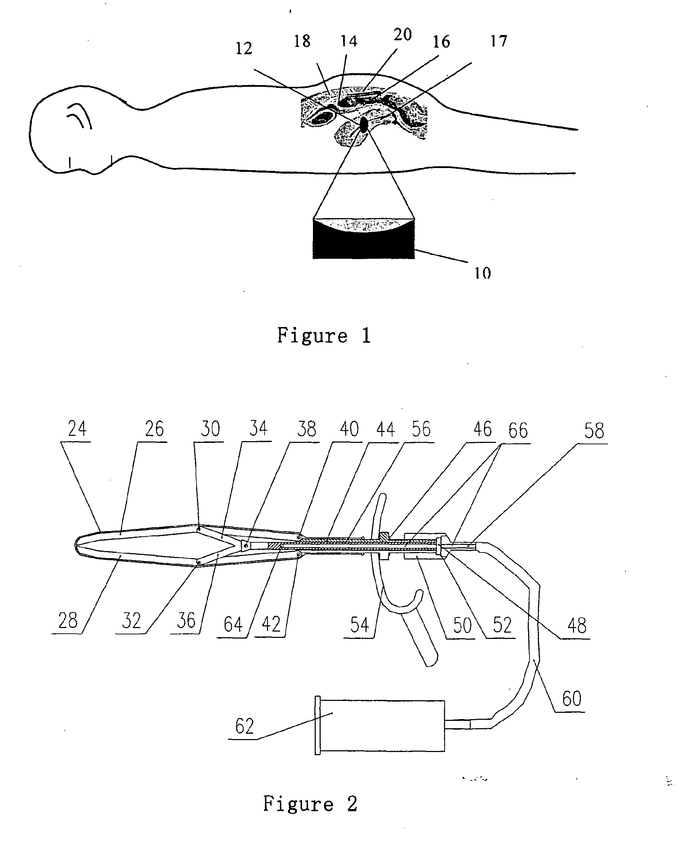

In order to better understand the present invention, Fig. 1 illustrates the existing high-intensity focused ultrasound therapy applied to uterine myoma without application of the ultrasound blocking apparatus within body. Ultrasonic source **10** generates ultrasound waves, which are focused at the target area of uterus **17.** Then a focal point **12** is formed. If there is not any air in rectum **14,** the ultrasound waves passed through the focal point continue to go along the ultrasound transmission path **16** and can reflect on the surface of spinal column **18** and a lesion point **20** is formed. Comparing to the existing technologies, the technical solutions for removing the air in the rectum and eliminating the unexpected lesion point by use of the apparatus disclosed in the present invention are described thereinafter in detail.

Fig. 2 shows an apparatus for delivering ultrasound blocker into the rectum of a patient of the present invention. It comprises (1) lead-in means for entering into the body cavity of mammals; (2) accommodation means for containing the medium, which enwraps the lead-in means outside and is used to block the ultrasound.

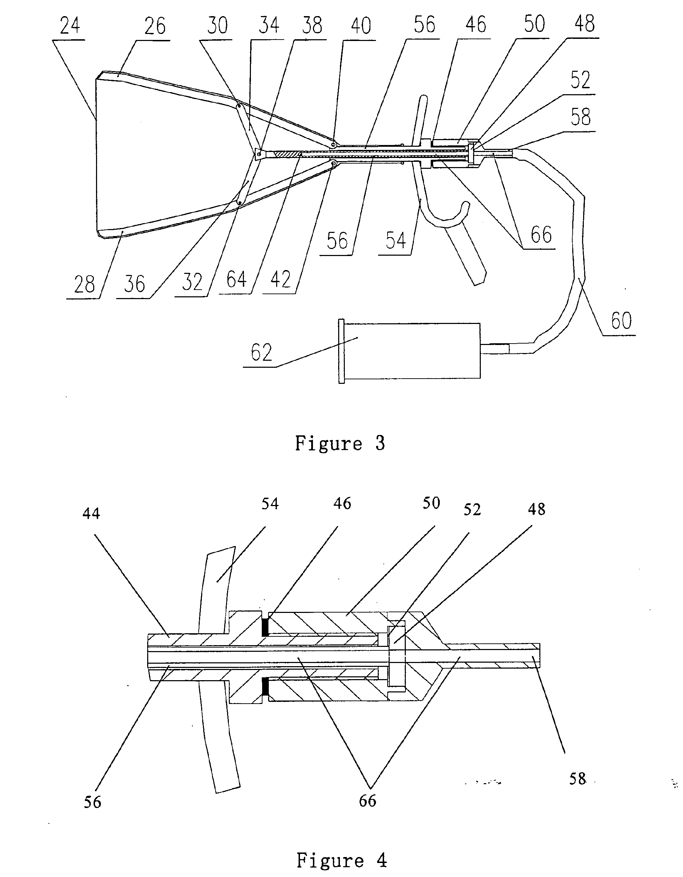

Fig. 2 and Fig. 3 show the structure and operation of the lead-in means of the embodiment. Expansion arms **26, 28** are respectively connected to expansion rods **34, 36** through pivots **30, 32.** Expansion arms **26, 28** are also respectively connected to fixture **44** through pivots **40, 42.** Another ends of expansion rods **34, 36** are connected to expansion link **56** through pivot **38.** The fixture **44** is connected to handle **54** with a rigid connection. The distal end of the fixture **44** has external screw threads. The push component **50** has internal screw threads matching with the screw threads of the fixture **44.** When the push component **50** rotates along the external screw threads of the fixture **44,** it can move relative to the fixture **44.** The movement limit is decided by the length of the said matching screw threads. The distal end of the push component **50** is closed and has a lumen **52,** in which a moving block **48** is accommodated. There is a gap between the moving block **48** and the lumen **52** so that the rotation of the push component **50** can not drive the moving block **48** to rotate. The movement of the push component **50** relative to the fixture **44** drives the moving block **48** to axially move forward (to the left in figure ) or backward (to the right in figure). The moving block **48** is connected to the expansion link **56** in a rigid connection. It can drive the expansion link **56** to axially move along the fixture **44.** The expansion link **56** moves forward (to the left in figure) to make the expansion rods **34, 36** expand relatively and drive the expansion arms **26, 28** to expand correspondingly. The expansion link **56** moves backward to make the expansion rods **34, 36** close relatively and drive the expansion arms **26, 28** to close correspondingly. The degree of movement relative to the fixture 44 of the push component **50** can control the expansion degree of the expansion arms **26, 28.** The lead-in means adopting four-bar linkages with functions of expanding and closing is described as above.

Under the closing status, the lead-in means is in a shuttle-shape and its front end is closed so that it can be inserted into the rectum of the patient to be treated in a gradual way. The material for the insertion part of the lead-in means is preferably selected from polymer materials. Particularly, when it is applied to the ultrasound therapy, the inappropriate reflection or focusing of ultrasound waves within body should be avoided. If that apparatus is used for other purposes, the material of the apparatus shall be selected correspondingly.

Accommodation means **24** enwraps the expansion arms **26, 28** and the fixture **44.** The distal end of the accommodation means **24** is fixed on the fixture **44** close to the handle **54.** Accommodation means **24** may be integrated with the lead-in means in a fixed connection. So this integrated device preferably is an integrated device for a single use. Also, the accommodation means may be fixed with the lead-in means just before use. Thus, only the accommodation means is for a single use, while the lead-in means can be used for many times, accordingly the cost is reduced. The fixing methods have many choices including binding with strips, fixing with adhesive tape or placing the flexible flange interface of the accommodation means itself into the corresponding grooves of the lead-in means.

The accommodation means is made of flexible material. Its surface contacts with the wall of body cavity. It is only used for a short time during treatment or check. Therefore, the material of the accommodation means shall have appropriate biocompatibility. According to the specific needs, some lubricating agent or coupling agent may be applied to the surface of the accommodation means so that the device can be inserted into the body more easily and it can fully contacts with body cavity wall. The material of the accommodation means is preferably selected from the flexible material. Because the vesicle made of flexible material is more adaptive to enwrap the lead-in means closely and it is easily carried into the body cavity by the lead-in means. In the embodiments, this accommodation means is a latex vesicle with an appropriate shape.

The lead-in means and accommodation means may be designed in different dimensions so as to be adaptive to the different dimensions of the body cavities to be occupied of different subjects to be treated For example, the dimensions of different apparatuses, which are placed in the rectum, are selected according to the body height. Normally, the length of the lead-in means that placed in the rectum is 100mm-160mm and the expanding width is 50mm-150mm.

The operator can hold the handle **54** under the closing status of the expansion arms **26, 28** and then push the lead-in means lightly into the rectum of the patient. Rotating the push component **50** makes the expansion arms **26, 28** expand and the degree of expansion can be controlled.

There is a lumen within the expansion link **56** and there is at least one outlet **64** for medium at the front end close to the lumen and also both the moving block **48** and the push component **50** have holes. These holes form a medium passageway **66.** The medium passageway **66** is connected to the hose **60.** The other end of the hose **60** is connected to the infusion and storing means **62.**

The infusion and storing means **62** may be the structure of injection means as shown by figure. Through pushing the plungers, the medium is delivered along the way from the hose **60,** the medium passageway **66,** the medium infusion port **58** to the lumen of the expansion rod **56,** then from the medium outlet **64** at the end of the lumen of the expansion link to the accommodation means **24.** The occupation of medium in the rectum and the expansion of the rectum can be controlled by controlling the total amount of the medium infused or observing by B-mode scanner.

As shown in Fig.3, the expansion arms **26, 28** keep the open status and the rectum is racket-shaped correspondingly. Even through the accommodation means **24** is full of medium, the relative position of the rectum keeps relatively flat. The flat part of the rectum is full of ultrasound attenuating medium, which occupies the space of air in the rectum so as to solve the exhaust problem. Meanwhile, that flat-shaped rectum may become a block for continuous ultrasound transmission behind the therapeutic focal point and the ultrasound attenuating medium absorbs the ultrasound energy and accordingly the lesion point on the surface of the spinal column as shown in Fig. 1 can be eliminated.

The ultrasound attenuating medium used in the present invention shall have biocompatibility so that the harm to the patient due to the disrepair of the accommodation means during the use can be prevented. The oil is the ideal ultrasound attenuating agent. The ultrasound attenuating agents used in the present invention include but are not limited to castor oil, silicone oil, soybean oil and peanut oil. The castor oil with a low cost is selected preferably in the present invention.

The simple mechanical structures of the present invention are easily modified or substituted by other existing technologies. The technicians skilled in the art may easily make numerous changes and improvements of the embodiments described as above or make it apply to other fields. This invention includes all kinds of embodiments and applications. Even through this invention is described according to the preferred embodiments, therefore the scope of the invention is not to be restricted, except by the following claims of this invention.

### The list of marking numbers in the drawings

- 10: Ultrasonic source
- 12: Focal point
- 14: Rectum
- 16: Ultrasound transmission path behind focal point
- 17: Uterus
- 18: Spinal column
- 20: Lesion point
- 24: Accommodation means
- 26, 28: Expansion arms
- 30, 32: Pivots
- 34, 36: Expansion rods
- 38: Pivot
- 40, 42: Pivots
- 44: Fixture
- 46: Seal
- 48: Moving block
- 50: Push component
- 52: Lumen
- 54: Handle
- 56: Expansion link
- 58: Medium infusion port
- 60: Hose
- 62: Infusion and storing means
- 64: medium outlet of expansion link lumen
- 66: Medium passageway

## Claims

1. An apparatus for delivering a medium to body cavity of mammals, wherein said apparatus comprising:
(1) lead-in means for entering into said body cavity; and
(2) accommodation means for containing said medium, which enwraps the lead-in means outside.

2. Apparatus as claimed in claim 1 wherein said lead-in means can expand and close.

3. Apparatus as claimed in claim 2 wherein said lead-in means adopts a connecting rod structure to realize said expanding and closing.

4. Apparatus as claimed in claim 3 wherein said connecting rod structure of lead-in means adopts nuts and screws and is operated manually.

5. Apparatus as claimed in claim 4 wherein further comprising a medium delivering means.

6. Apparatus as claimed in claim 5 wherein further comprising a medium storing means for storing said medium before use.

7. Apparatus as claimed in any of claims 1-6 wherein said accommodation means is made of flexible material.

8. Apparatus as claimed in claim 7 wherein said medium can be selected from the group consisting of blocker, absorbing and attenuating agent.

9. Apparatus as claimed in claim 8 wherein said ultrasound attenuating agent is selected from the group consisting of free castor oil, silicone oil, soybean oil and peanut oil.

10. Apparatus as claimed in claim 9 wherein said body cavity of mammals is rectum.

11. An apparatus for expanding a body cavity of mammal, wherein a lead-in means with a connecting rod structure is adopted to realize expanding and closing.

12. Apparatus as claimed in claim 11 wherein said body cavity of mammals is rectum.

13. Apparatus as claimed in claim 12 wherein the nuts and screws are adopted to manually operate said connecting rod structure so as to realize expanding and closing.

14. Apparatus as claimed in claim 13 wherein it is used to lead an accommodation means into said body cavity.

15. An apparatus for blocking ultrasound, wherein said apparatus comprising,
(1) lead-in means for entering into said body cavity;
(2) accommodation means for containing ultrasound attenuating medium, which enwraps the lead-in means outside.

16. Apparatus as claimed in claim 15 wherein said accommodation means is a vesicle made of flexible material, which enwraps said lead-in means outside.

17. Apparatus as claimed in claim 16 wherein said lead-in means can expand and close.

18. Apparatus as claimed in claim 17 wherein said lead-in means adopts a connecting rod structure to realize said expanding and closing.

19. Apparatus as claimed in claim 18 wherein said body cavity of mammals is rectum.

20. Apparatus as claimed in claim 19 wherein said connecting rod structure of lead-in means adopts nuts and screws for manual operations.

## Amended claims

### Amended claims under Art. 19.1 PCT

**1.** An apparatus for delivering a medium to body cavity of mammals, wherein said apparatus comprising:
(1) lead-in means for entering into said body cavity; and
(2) accommodation means for containing said medium, which enwraps the lead-in means outside,
wherein said lead-in means can expand and close.

**2.** Apparatus as claimed in claim 1 wherein said accommodation means is a vesicle made of flexible material.

**3.** Apparatus as claimed in claim 2 wherein said lead in means adopts a connecting rod structure to realize said expanding and closing.

**4.** Apparatus as claimed in claim 3 wherein said connecting rod structure of lead-in means adopts nuts and screws and is operated manually.

**5.** Apparatus as claimed in claim 4 wherein further comprising a medium delivering means.

**6.** Apparatus as claimed in claim 5 wherein when said lead-in means in open, it can make said accommodation means, which enwraps the lead-in means outside, become racket-shaped.

**7.** Apparatus as claimed in claim 6 wherein further comprising a medium storing means for storing said medium before use.

**8.** Apparatus as claimed in claim 6 wherein said medium can be selected from the group consisting of blocker, absorbing and ultrasound attenuating agent.

**9.** Apparatus as claimed in claim 8 wherein said ultrasound attenuating agent is selected from the group consisting of free castor oil, silicone oil, soybean oil and peanut oil.

**10.** Apparatus as claimed in claim 9 wherein said body cavity of mammals is rectum.

**11.** Apparatus as claimed in any of claims 2-10 wherein said vesicle made of flexible material is latex vesicle.

**12.** An apparatus for expanding a body cavity of mammal, wherein a lead-in means with a connecting rod structure is adopted to realize expanding and closing.

**13.** Apparatus as claimed in claim 12 wherein the nuts and screws are adopted to manually operate said connecting rod structure so as to realize expanding and closing.

**14.** Apparatus as claimed in claim 13 wherein it is used to lead an accommodation means into said body cavity under the closing status, and make said accommodation means have a specific shape making use of the expanding status of said lead-in means.

**15.** Apparatus as claimed in claim 14 wherein said shape of said accommodation means under said expanding status is racket shape.

**16.** A ultrasound blocking apparatus applied to ultrasound trerapy, wherein said apparatus comprising,
(1) lead-in means for entering into rectum of mammals, wherein said lead-in means can expand and close;
(2) accommodation means, made of flexible material, for containing ultrasound attenuating medium, which enwraps the lead-in means outside.

**17.** Apparatus as claimed in claim 16 wherein said lead-in means adopts a connecting rod structure to realize said expanding and closing.

**18.** Apparatus as claimed in claim 17 wherein said connecting rod structure of lead-in means adopts nuts and screws for manual operations.

**19.** A method of providing protection to patients who receive high-intensity ultrasound therapy, wherein said method comprising the following steps, a ultrasound blocking apparatus is led into rectum; wherein said apparatus comprising lead-in means and accommodation means, and said accommodation means for containing ultrasound attenuating medium, which enwraps the lead-in means outside.

**20.** Method as claimed in claim 19 further comprising the steps of making said lead-in means expanding and making said accommodation means become racket-shaped.
